# EUROPEAN PATENT SPECIFICATION

(11) **EP 3 797 418 B1**
(45) Date of publication and mention of the grant of the patent: **03.07.2024**
(21) Application number: 19734884.0
(22) Date of filing: 16.05.2019
(51) Int. Cl.: G16B 30/00, G16B 99/00, A61B 5/00

(54) **METHOD FOR DETERMINING THE PROBABILITY OF THE RISK OF CHROMOSOMAL AND GENETIC DISORDERS FROM FREE DNA OF FETAL ORIGIN**
VERFAHREN ZUR BESTIMMUNG DER WAHRSCHEINLICHKEIT DES RISIKOS VON CHROMOSOMALEN UND GENETISCHEN STÖRUNGEN AUS FREIER DNA FETALEN URSPRUNGS
PROCÉDÉ DE DÉTERMINATION DE LA PROBABILITÉ DE RISQUE DE TROUBLES CHROMOSOMIQUES ET GÉNÉTIQUES À PARTIR D'ADN LIBRE D'ORIGINE FOETALE

(30) Priority: 23.05.2018 IT 201800005623
(43) Date of publication of application: 31.03.2021
(73) Proprietor: Artemisia S.p.A., 00198 Roma (RM) (IT)
(72) Inventor: DELLO RUSSO, Claudio, 00198 ROMA(RM) (IT); GIORLANDINO, Claudio, 00198 ROMA (RM) (IT); MESORACA, Alvaro, 00198 ROMA (RM) (IT)
(74) Representative: Fiammenghi, Eva
(86) International application number: PCT/IB2019/054068
(87) International publication number: WO 2019/224668

(56) References cited:
- US-A1- 2016 224 724
- US-A1- 2016 281 166
- BARAN BAYINDIR ET AL: "Noninvasive prenatal testing using a novel analysis pipeline to screen for all autosomal fetal aneuploidies improves pregnancy management", EUROPEAN JOURNAL OF HUMAN GENETICS., vol. 23, no. 10, 14 January 2015 (2015-01-14), pages 1286-1293, XP055378014, CH ISSN: 1018-4813, DOI: 10.1038/ejhg.2014.282
- JAMES K. BONFIELD ET AL: "Compression of FASTQ and SAM Format Sequencing Data", PLOS ONE, vol. 8, no. 3, 1 January 2013 (2013-01-01) , page e59190, XP055330942, DOI: 10.1371/journal.pone.0059190
- A. J. COX ET AL: "Large-scale compression of genomic sequence databases with the Burrows-Wheeler transform", BIOINFORMATICS., vol. 28, no. 11, 3 May 2012 (2012-05-03), pages 1415-1419, XP055544651, GB ISSN: 1367-4803, DOI: 10.1093/bioinformatics/bts173
- LA VERDE MARCO ET AL: "Performance of cell-free DNA sequencing-based non-invasive prenatal testing: experience on 36,456 singleton and multiple pregnancies", BMC MEDICAL GENOMICS, vol. 14, no. 1, 30 December 2021 (2021-12-30), XP093126676, London UK ISSN: 1755-8794, DOI: 10.1186/s12920-021-00941-y

## Description

### Field of the invention

The present invention relates to a method for determining the probability of the risk of chromosomal and genetic disorders from free DNA of fetal origin.

In particular, the present invention relates to a method for determining the probability of anomaly risk on the chromosome of interest for determining the probability of microdeletion or microduplication risk on the genomic regions that make up the genome.

### Prior art

Prenatal tests, including prenatal screening and prenatal diagnosis, allow the detection of problems related to the anatomical and physiological development of the zygote, embryo or fetus in the initial phase of gestation.

Among the methods of prenatal screening, of the non-invasive type, a method of blood analysis is known, based on the detection of free DNA of fetal origin from cells present in the maternal blood, or cffDNA "cell-free fetal DNA", defined as *"Non-invasive Prenatal Test"* (NIPT). Reading fetal DNA, in addition to revealing the sex of the unborn child, allows us to recognize the presence of chromosomal anomalies (errors in the number of chromosomes), in particular for chromosome 21 (Down syndrome), and for other more common trisomies (chromosomes 13, Patau, and 18, Edwards). The NIPT is a predictive and non-diagnostic test able to identify women at high risk of having a child with chromosomal diseases whose possible positive result requires prenatal diagnostic tests of the invasive type, such as amniocentesis or villocentesis. The NIPT is a test which can be performed starting from the tenth week of gestation and has a high reliability but also a high variance in the results connected to the plurality of tests on the market.

However, the NIPT exhibits a plurality of limits, mainly related to non-high sensitivity (identification of about 50% of the anomalies routinely identified with prenatal diagnostic tests of the invasive type), impossibility of distinguishing all aneuploidies, presence of false positives and negatives (between 0.1% and 0.5%), need for an adequate amount of fetal DNA in maternal blood (over 5%), as well as the impossibility of distinguishing the condition of the single fetus in the case of twin pregnancy.

From the first weeks of pregnancy it is possible to detect in the maternal blood circulation the presence of intact fetal cells and free DNA of fetal origin, which constitutes a variable fraction generally comprised between 3 and 20% of the total extracellular DNA detectable in the maternal circulation, with a concentration that tends to progressively increase during pregnancy and can vary in the presence of fetal aneuploidies, in particular decreasing in the presence of trisomy 13 and 18 and increasing in the presence of trisomy 21. Free DNA of fetal origin is present in the form of fragments of reduced size compared to those that constitute the maternal fraction. The placenta and, in particular, apoptotic trophoblast syncytium cells, are the main source of cffDNA, which is then completely removed from the maternal circulation, probably through renal excretion, within a few hours of delivery.

A further critical point of the cffDNA analysis is that it represents, on average, 10% of the total DNA extracted from the plasma, while the predominant fraction is represented by the maternal DNA. It is however possible to perform the prenatal screening test of the most common fetal aneuploidies by using high sensitivity technologies such as digital PCR, Massively Parallel Sequencing (MPS), SNP-based NGS, Digital Analysis of Selected Regions (DANSR).

The result of the non-invasive type test is also conditioned by the percentage of fetal DNA present in the plasma which must be higher than 3-4%, since lower quantities may result in false negative results. The relative amount of fetal DNA is reduced in particular conditions such as gestational age less than 10th week and a high body mass index.

US2016/224724 discloses methods and devices for a non invasive assessment of genetic variations in a fetus and is considered to be the closest prior art..

### Summary of the invention

The object of the present invention is to provide a non-invasive prenatal screening method for calculating the probabilities of risk of genetic and chromosomal anomalies found in the fetus. Said method is implemented thanks to a peculiar software that offers a higher precision of the obtainable results compared to the NIPT methodologies currently available for patients.

The above objects are achieved by a method for determining the probability of the risk of chromosomal and genetic disorders from free DNA of fetal origin according to the appended claims.

### Description of the invention

The present invention relates to a method of screening and analysis of fragments of free DNA circulating in the maternal blood, called fetal free DNA or ffDNA, deriving from the trophoblast (the structure that forms the placenta), which, in the vast majority of cases, follow the composition of fetal DNA. In particular, the present invention relates to a method for determining the probability of the risk of chromosomal and genetic disorders from free DNA of fetal origin.

More specifically, the method according to the present invention is a screening method capable of assessing the risk of the fetus being a carrier of chromosomal anomalies. The method according to the present invention is able to obtain a very low number of false negatives and false positives, lower than 0.1%, compared to the known methods which, instead, have values ranging from 0.1% to 0.3% of cases.

According to the present invention, the method for screening and analyzing the fetal DNA present in a sample of maternal plasma previously taken, uses a technology for the preparation of DNA libraries to verify the presence of chromosome anomalies. DNA isolated from the maternal blood sample includes both maternal and fetal genetic material. The cffDNA is then isolated and the relative quantity of genetic material that aligns with the chromosomes studied is analyzed.

The method for determining the probability of the risk of chromosomal and genetic disorders from free DNA of fetal origin, according to the present invention, comprising a plurality of steps, described below.

First there is the step of extracting the free DNA of fetal origin, that is, the cffDNA, from a sample of maternal plasma previously taken, preferably contained in suitable test tubes.

An automated protocol for total DNA purification from fetal blood, isolated from maternal blood samples, may be used. For example, magnetic particle technology may be used to purify high quality nucleic acids that are free of proteins, nucleases and other impurities. Purified nucleic acids are subsequently and directly used in downstream applications (such as DNA library preparation).

A step of sequencing the free DNA of fetal origin is then carried out.

The DNA extracted from the maternal plasma sample is added to a final repair reaction which includes incubating the DNA sample with an enzymatic mix. The fragments are phosphorylated at the 5' end to allow subsequent ligation with the adapter oligonucleotides. The reaction ends with an incubation step to denature final repair enzymes. At the end of the final repair reaction, the ligation reaction with the adapters is obtained. The ligation reaction with the adapters includes an incubation of the sample with oligonucleotides. These enzymes bind the adapter oligonucleotides to DNA fragments and repair the junction between the DNA fragment and the bound adapter. At the end of the ligation reaction with the adapters, a cleaning step is performed using the reagents in paramagnetic spheres. Paramagnetic spheres are added to the sample attached to the adapters, to join the DNA fragments bound to the adapters and isolate them from the unused adapters. The spheres to which the DNA fragments are bound are separated from the solution by means of a magnet and washed with nuclease-free water before DNA elution.

After the cleaning step it is possible to carry out a step of amplification of free DNA of fetal origin by means of PCR, Polymerase Chain Reaction, where the DNA fragments bound to the adapters, now isolated, can be subjected to the so-called PCR amplification. The aforementioned amplification step is carried out downstream of the step of extraction of the free DNA of fetal origin and upstream of the step of sequencing of said free DNA of fetal origin extracted.

The terms "Chain Polymerase Reaction" or "PCR" mean, in the present invention, the techniques of molecular biology which allow the multiplication (amplification) of fragments of nucleic acids of which the nucleotide initial and final sequences are known. PCR amplification allows the amount of genetic material needed for subsequent applications to be obtained very quickly in vitro.

In particular, the DNA fragments obtained in the cleaning step are subjected to PCR amplification with PCR reagents. To generate the amplified DNA library, a PCR mix is used containing high-fidelity DNA polymerase enzyme and PCR primers designed to bind sequences in adapter oligonucleotides.

The DNA libraries are cleaned with the reagents in paramagnetic spheres. The paramagnetic spheres are added to the DNA libraries to join the DNA fragments bound to the adapters and amplified and to isolate them from the reaction mixture and the unused PCR primers.

The step of selecting the size of the pool of DNA libraries subjected to multiplexing takes place using the reagents in paramagnetic spheres. DNA libraries are selected based on size, so that the fragments fall within the reading length range suitable for the sequencing platform. First, the paramagnetic spheres are added to the pool of multiplexed DNA libraries to bind any larger undesirable fragments present in the sample. The spheres to which the DNA fragments are bound are separated from the solution by means of a magnet and the supernatant containing the DNA library fragments of the desired size is kept. The paramagnetic spheres are then added to the supernatant to bind the remaining fragments of selected DNA libraries based on their size.

The spheres are separated from the solution using a magnet and washed with nuclease-free water before DNA elution. The pool of DNA libraries subjected to multiplexing and selected on the basis of size is quantified and diluted to obtain the suitable concentration for the sequencing reaction.

The prepared sample is then placed in a sequencing platform. The enzymatic mix contains the enzymes DNA ligase and DNA polymerase. Such sequencing is preferably carried out by Next Generation Sequencing NGS techniques, but may likewise be achieved by single sequencing techniques.

The terms "Next Generation Sequencing" or "NGS" or "Parallel sequencing" refer, in the present invention, to the technologies that allow sequencing large genomes in a short time, preferably in the order of weeks.

Sequencing with NGS multiplexing technology is therefore performed according to the protocol for the relevant sequencing platform with NGS technology. Following the sequencing of multiplexed DNA libraries, the data obtained are processed by suitable software which calculates the probability that the fetus may be affected or not by one of the examined trisomies, fetal aneuploidy or for the study of microdeletions or microduplication syndromes.

Non-invasive prenatal analysis (also known as NIPT, Non-Invasive Prenatal Testing) uses cfDNA (cell-free DNA) extracted from maternal peripheral blood to investigate genetic disorders in the fetus. In contrast to invasive methods, such as amniocentesis, the NIPT system does not present risks of miscarriage.

To perform the NIPT analysis, cfDNA is extracted from maternal plasma and sequenced using an NGS (Next Generation Sequencing) system.

Therefore, the step of storing the sequencing carried out in FASTQ format, comprising the biological sequences and the quality scores of the biological sequences themselves, follows. In particular, after sequencing the machines return a file, called FASTQ, which contains the storage of all the sequences obtained and the respective quality scores.

The term "FASTQ format" means, in the present invention, a text-based format for storing both a biological sequence (usually the nucleotide sequence) and the relative quality scores. Both the sequence letter and the quality score are each encoded with a single ASCII character for brevity.

The FASTQ file therefore contains information on the quality of sequencing, in particular it contains information on the Phred quality score, or the measurement of the quality of the identification of nucleobases generated automatically by DNA sequencing. The Phread quality score is calculated as: Q = -10log(P).

In the next step, the sequencing conversion from the FASTQ format to the SAM format is performed, in which the biological sequences are aligned with a reference genome.

The terms "Sequence Alignment Map" or "SAM" mean, in the present invention, a text-based format for storing biological sequences aligned to a reference sequence. This format is used for storing data, such as nucleotide sequences, generated by NGS technologies. The SAM format is used to contain data mapped within the Genome Analysis Toolkit (GATK) and consists of a header and an alignment section.

According to the step of converting the sequencing from the FASTQ format to the SAM format, in which the biological sequences are aligned with a reference genome, it is implemented using the Burrows-Wheeler algorithm.

The application of the aforementioned algorithm can be implemented, for example, by means of the Bowtie2 software package, preferably on a Linux/Unix platform. The aforementioned packages and platform are not, however, binding. Bowtie2 is an ultra-fast and efficient tool for aligning reads (or sequencing fragments produced by NGS systems) to a reference genome, as described in more detail below. Bowtie2 indexes a genome using the Burrows-Wheeler algorithm, which is the gold standard for NGS sequence alignments.

To construct the reference, thanks to which the reads will be aligned, it will be necessary to have the reference genome, that is, the assembly sequence for each chromosome in a single file, to allow all the assembly files to be condensed into a single file, or the reference genome to be used according to the present invention.

The term "READ" means, in the present invention, a small sequence of synthesis DNA obtained from a sequencing reaction. First the DNA of the patient is broken into many small fragments, to each of which short nucleotidic sequences (so-called adapters) are added which serve to anchor the DNA fragments to the support on which the sequencing reaction will take place. The set of DNA fragments prepared by the addition of adapters constitutes the sequencing library. The sequencing library fragments are amplified and sequenced to obtain numerous complementary copies of each fragment, defined as reads.

The actual reference index for alignment is therefore created, with the production of six files that will constitute the index to which the software for the application of the method according to the present invention will refer. After the construction of the index, the reads contained in the FASTQ files produced by the NGS sequencing of DNA samples extracted from the maternal plasma will be aligned.

Alignment is the process by which one discovers how and where the reading sequences are similar to the reference sequence, that is, aligning some or all the characters present in the reads with the reference ones.

In a further conversion step, sequencing is converted from the SAM format to the BAM format, in which the data contained in the SAM format are compressed by conversion into binary code.

The terms "Binary Alignment Map" or "BAM" mean, in the present invention, the compressed and lossless binary representation of the Sequence Alignment Map.

The conversion from the SAM format to the BAM format is essential to improve the performance of all the other software packages that will be used for the method according to the present invention.

The method according to the present invention may further comprise the step of defining an index BAI format from said compressed BAM format, wherein the BAI index format is suitable for the execution of every software package that works on a compressed BAM format.

At this point, it is possible to carry out the steps of the method for the actual execution of the NIPT analysis.

With the method according to the present invention it is possible to carry out the above analysis on chromosomes 13, 18 and 21 for the determination of trisomies of the aforementioned chromosomes and/or for the determination of fetal sex and aneuploidy of sex chromosomes X, Y and/or for the determination of the trisomies of said autosomes relative to one or more of the chromosomes 1, 2, 3, 4, 5, 6, 7, 8, 9, 10, 11, 12, 14, 15, 16, 17, 19, 20, 22. Such tests may, for example, be carried out in the z-score calculation step, as described in greater detail below, on a chromosome of interest with respect to the predetermined reference sample.

Moreover, with the method according to the present invention it is possible to carry out the aforementioned analysis for the determination of one or more of Wolf-Hirschoorn Syndrome, Jacobsen Syndrome, 1p36 deletion syndrome, Angelman Syndrome, DiGeorge Syndrome, Cri-du-chat Syndrome, Langer-Giedion Syndrome, Smith-Magenis Syndrome, Prader-Willi Syndrome, Williams Syndrome, Koolen-de-Vries Syndrome, HNPP Syndrome, 18q deletion syndrome, WAGR Syndrome, Potocki-Shaffer Syndrome, 1q21.1 deletion syndrome, Kleefstra, Phelan-Mcdermid Syndrome, Rubistein-Taybi Syndrome, Alagille Syndrome and Miller-Dieker Syndrome. Such tests may, for example, be carried out in the step of determining the probability of risk of microdeletion or microduplication, as described in greater detail below, on the TILES genomic regions that make up the genome.

The invention provides a further method for determining the probability of the risk of chromosomal and genetic disorders from free DNA of fetal origin, wherein the determination of the probability of risk of:
1. Cystic fibrosis (CFTR GENE)
2. Congenital deafness (GJB2 GENE)
3. Beta thalassemia (HBB GENE)
4. Classical congenital adrenal hyperplasia due to 21-hydroxylase deficiency (CYP21A2 GENE)
5. Hemochromatosis (HFE GENE)
6. Achondroplasia (FGFR3 GENE)
7. Hypochondroplasia (FGFR3 GENE)
8. Dysatophor dysplasia (FGFR3 GENE)
9. Apert syndrome (FGFR2 GENE)
10. Crouzon syndrome (FGFR2 GENE)
11. Leopard syndrome (PTPN11 GENE)
12. Noonan syndrome (PTPN11/SOS1/RAF1 GENES)
13. Phenylketonuria (PAH GENE)
14. Rett syndrome (MECP2 GENE)
15. Recessive polycystic kidney (PKHD1 GENE) is obtained.

The invention also provides for the study of the risk of preterm birth which is now the most frequent cause of neonatal fetal mortality and morbidity. It is a study that is conducted by examining a series of genetic susceptibility polymorphisms (SNP) (see Patent application no. 102017000134611 of 11/23/2017).

The invention also provides the study, in the maternal blood, in the first three months, of the cytomegalic and toxoplasmic infection. Maternal cytomegalic infection tends to be asymptomatic and patients are rarely diagnosed with clinical symptoms alone. For most infections, evidence of maternal seroconversion (defined as a conversion from negative to positive IgM or a 4-fold increase in IgG antibody titer over 4-6 weeks) is sufficient to confirm the diagnosis of a primary infection. However, the accuracy of maternal anti-CMV IgM to predict primary maternal infection is complicated by the fact that IgM antibodies can persist for months or even years after primary infection, and can also be found in the reactivation or reinfection setting with a different strain of CMV.12.

Another method for determining the timing of maternal CMV infection is to measure the avidity of the antibody, which refers to the strength of the binding of the antibody to a target antigen. While the immune response to a particular antigen matures over time, avidity increases. Thus, detection of low-avidity anti-CMV IgG early in pregnancy suggests a recent acute infection and may be used to identify pregnant women at increased risk of having an infected fetus. In contrast, the presence of high avidity antibodies at 12-16 weeks of gestation indicates a past infection, probably before conception.

The best method therefore is the direct search for the virus in the maternal blood (possibly completed with research in the urine). This is why the Fetal-maternal total screen includes this fundamental exam for managing pregnancy.

For maternal toxoplasmic infection, knowing the presence of an infection in the maternal compartment early allows establishing an adequate therapy with a true and absolute therapeutic rationale.

All these tests can be performed by implementing the method according to the present invention with suitable software packages preferably implemented on statistical software. Some factors influence the performance of the analysis, for example the fetal fraction, or the proportion between reads obtained from cfDNA sequencing with respect to maternal DNA, the length of the reads, and the coverage, i.e. the coverage of the reads with respect to the reference genome.

Then, the step of counting and categorizing the READ sequenced from the SAM format follows, in which each READ is categorized as mapped on each chromosome or mapped on all autosomes.

Appropriate software packages can be used to analyze BAM files, count reads and categorize them. Unprocessed reads from high-throughput sequencing technologies generally do not follow theoretical counting statistics. The excess variance, produced in part by PCR steps, is related to the GC content of the DNA sequence. This excess variance is normalized by the NCV normalized chromosome value method and by the count weighting by category of the GC content. The NCV calculates the ratio between the counts mapped on the chromosome of interest and another chromosome, while for the weighting of the counts by category in GC, the count of the reads first is determined for each category, then the average count is calculated as a function of the content in GC. It is also possible, through this analysis, to mask the repeated and low complexity areas of the genome, so as to take into account only the uniquely mapped reads.

Subsequently, the step of calculating the ratio between the number of READ mapped on each chromosome and the number of READ mapped on all autosomes is carried out. For each sample, the ratio of the reads mapped on each chromosome to the reads mapped on all autosomes is calculated.

After the removal of the artifacts from the reads, in the step of defining a predetermined reference sample, a reference is created using a series of reference samples with known results and fetal sex.

Then, in the step of calculating the z-score on a chromosome of interest with respect to the predetermined reference sample. For example, the z-score on chromosomes 13, 18 and 21 can be calculated.

Finally, in a further step, the probability of anomaly risk on the chromosome of interest is determined if the calculated z-score is higher than a predetermined value. Typically, in the case of z-scores with a value greater than three, a chromosomal anomaly is declared on the analyzed chromosomes.

For the determination of fetal sex and monosomy X, instead, the z-score will be calculated for the X chromosome and for the Y chromosome and, as described above, if the calculated z-score value is greater than 3 for the Y chromosome, the sample will be declared male fetal sex, while if it is greater than -2 for the X chromosome it will be declared female fetal sex.

Finally, the method according to the present invention further comprises the step of verifying the free DNA of fetal origin by DigitalPCR if in the step of determining the probability of anomaly risk on the chromosome of interest the predetermined value is exceeded by said z-score.

The digital polymerase chain reaction (Digital PCR, dPCR) is a biotechnological improvement of conventional polymerase chain reaction methods that can be used to directly quantify and amplify nucleic acid filaments. The dPCR quantitatively measures a certain variable and also performs a single reaction within a sample that is separated into a large number of partitions and the reaction is performed individually in each partition. This separation allows for more reliable collection and a sensitive measurement of nucleic acid quantities. This precision makes the verification of a positive test by NGS even more reliable, increasing the sensitivity of the method in question and decreasing the share of false positives.

DigitalPCR is, therefore, an application in the detection and quantitative analysis of small units of DNA, providing a highly sensitive quantitative analysis and detection of target nucleic acid molecules by simultaneously processing a different number of samples. Together with NGS sequencing, DigitalPCR was used to enhance the result coming from an NIPT test and significantly reduce the percentage of false positives.

The procedure for searching for microdeletions and microduplications is extremely similar to that illustrated for the search for chromosomal anomalies, according to what is described in greater detail below.

A step of defining a set of TILES genomic regions that make up the genome in BAM format, in which the data are compressed by conversion into binary code, is carried out. The use of tiles allows defining categories that have variable widths and therefore allow greater flexibility of the function.

In a subsequent step of defining a reference genomic region in BAM format, the data are compressed by conversion into binary code, in which the parameters for the realization of the BAM format of the reference genomic region are the same as the BAM format of the TILES genomic regions, in such a way as to eliminate any difference between the two files that would entail the presence of numerous cases of false positives.

Therefore, a step of comparing the BAM format of the TILES genomic regions with the BAM format of the reference genomic region is carried out.

Finally, a step of determining the probability of microdeletion or microduplication risk on the TILES genomic regions that make up the genome is carried out.

A further application of the method according to the present invention relates to the study of the CFTR gene on the gestational genomic DNA for cystic fibrosis screening. Cystic Fibrosis (CF) is a genetic, multi systemic autosomal recessive transmission disease in which the defective transport of chlorine through cell membranes causes dehydrated and viscous secretions with consequent thickening of mucus in the bronchi, thickening of the pancreatic juice and high chlorine levels in sweat. The organs frequently affected are the liver, the intestine, the reproductive system and the lungs where the particularly dense mucus leads to serious respiratory problems and consequent infections. Children with Cystic Fibrosis have two parents who are at least healthy (or heterozygous) carriers of a gene mutation and, in particular, between two healthy carriers there is a 25% chance that the fetus may be affected by the disease. To date, more than 1500 mutations of cystic fibrosis (CFTR) gene are recognized, many of them are extremely rare and others are still unknown. With the method according to the present invention it is possible to carry out the analysis of the maternal gene through a screening called 1st level which allows analyzing the most common and frequent mutations succeeding in identifying approximately 83% of carriers. The estimated frequency, in the Italian population, of healthy carriers (often unaware of being so) is 1 in 25 - 30, that of the born affected is 1 in 2500 - 3000.

The method therefore comprises the steps of extracting the maternal DNA, specific amplification of the CFTR gene by means of diagnostic KIT which allows the simultaneous detection of mutations of the gene encoding the CFTR protein using the Dot Blot methodology and automatic fragment sequencing as described above. The test allows the identification of the main variants of the CFTR (Cystic Fibrosis Transmembrane Conductance Regulator) protein using Dot Blot technology starting from DNA extracted from whole blood and spot and subsequent fragment analysis on an automatic sequencer. In each tube, specific and simultaneous amplification of all the searched mutations is performed. This complex methodology requires a rigorous application of the protocol.

Mutations analyzed: 711+1G-T, 621+1G-T, 1717-1G-A, 3849+10kbC-T, 2789+5G-A, G542X, G85E, G551D, R553X, N1303K, R117H, R1162X, L1077P, L1065P, W1282X, R347P, I507del, T338I, F508del, 1677delTA, 2183AA-G, S549R, Q552X, 852del22, R1066H, G1244E, 1259insA, D1152H, 711+5G-A, R1158X, 4382delA, 4016insT, A455E, 1706del117, 1502T, 3199del6, S912X.

In this case, in the risk determination step it is possible to attribute a high risk probability that the subject is a heterozygote or healthy carrier, when a mutation among the analyzed ones is found in the maternal CFTR gene, or negative, when none of the mutations among those analyzed is found in the maternal CFTR gene.

A further application of the method according to the present invention relates to the study of the SMN1 gene on the gestational genomic DNA for the screening of Spinal Muscular Atrophy. Spinal muscular atrophy (SMA) is a group of genetic diseases that cause weakness and decay in the voluntary muscles of infants and children and, more rarely, in adults. It is one of the most common genetic conditions affecting children. It is estimated that one in 6,000 children worldwide was born with SMA.

In more than 95 percent of cases, SMA is caused by the inadequate production of a protein called survival motor neuron (SMN) protein essential for motor neurons. SMN is produced by SMN1 and to a lesser extent by SMN2. These genes are on chromosome 5. Typically, people have two copies of the SMN1 gene and up to two copies of the SMN2 gene in each of their motor neuron cells. In people with spinal muscular atrophy, both copies of the SMN1 gene are altered or missing. Having additional copies (three or more) of the SMN2 gene is associated with a milder disease partially compensating for the missing SMN1. Rarely, SMA is caused by mutations in genes other than SMN (not chromosome 5).

The motor neurons are located in the anterior horn of the spinal cord and directly control the skeletal muscles of the body. Without an adequate SMN protein, the spinal cord motor neurons begin to contract and die. When this happens, the child's brain cannot control the voluntary muscles of the body, especially those in the arms and legs and in the head and neck. The muscles start to weaken and disperse. This affects movements such as walking, crawling, checking the head and neck, swallowing and breathing.

There are four different types of spinal muscular atrophy. The classification is determined by the stages of development that the child has hit at the time of the onset of the disease. Types I and II are the most common.

The types of spinal muscular atrophy (SMA) are:
Type 1 SMA (severe): this type is also called Werdnig-Hoffmann disease. It is the most severe and common type of SMA. It is usually evident at birth or in the first few months (0-6 months). Symptoms include floppy limbs and weak trunk movement. Children with this type usually have a very limited ability to move. They will also have difficulty eating and swallowing, keeping their heads high and breathing. Type 1 SMA progresses rapidly, with weakening of the muscles leading to frequent respiratory infections and usually death by age 2. Infants with SMA type 1 can never sit.
Type 2 SMA (intermediate): symptoms usually appear between 7 and 18 months. The rate of progression can vary greatly. The disease affects the child's legs more than his arms. Children with SMA type 2 can never stand. Respiratory infections are also common with this type of SMA. Life expectancy can vary from early childhood to adulthood, depending on the severity of the patient's condition.
Type 3 SMA (mild): this type of SMA is also called Kugelberg-Welander or juvenile spinal muscular atrophy. Symptoms may appear for the first time in a wide range of years, from 18 months to the beginning of adulthood. Patients with type 3 SMA can stand and walk, but may have difficulty getting up from a sitting position. They may also have mild muscle weakness and are at greater risk for respiratory infections. Most patients with type 3 SMA have a life expectancy close to normal.
Type 4 SMA (adult): the symptoms of this rare type of SMA do not usually emerge until the second or third decade of life. Patients with type 4 SMA can walk during adulthood, but usually experience progressively slow muscle weakness and other symptoms typical of SMA.

A further application of the method according to the present invention relates to the study of the main genes associated with hereditary thrombophilia (Leiden Factor V, Factor II, MTHFR) and of the risk of preeclampsia, on the genomic DNA of the pregnant woman.

Yet another application of the method according to the present invention relates to the study of fetal Rh. Prenatal determination of the fetal Rh genotype could lead to a substantial reduction in the use of anti-D immunoglobulin and the prevention of unnecessary exposure of pregnant women carrying a negative RhD fetus. The Rh blood group is one of the most complex and polymorphic blood groups known in humans. The RHD and RHCE homologous genes encode two distinct Rh proteins that are found on chromosome 1. These genes each have 10 exons and 9 introns and share the homology at 96.8% at the nucleotide level.

Approximately 50% of cases of maternal alloimmunization come from maternal anti-D (IgG) antibodies that cross the placenta and bind to RHD-positive fetal red blood cells that lead to their destruction following anemia and in some cases contribute to hydrops fetalis and intrauterine fetal death or neonatal death.

By determining the fetal Rh genotype, anti-D immunoglobulin can be intentionally administered for RhD Rh-negative pregnant women carrying positive Rh of the fetus. Another advantage of fetal RhD genotyping concerns women who are immunized against antigen D for various reasons and who have anti-D in serum.

The method according to the present invention therefore allows the genotyping of fetal RHD from cell-free fetal DNA (cffDNA) in the plasma of pregnant women. Fetal RhD genotyping is performed using the Real Time PCR technique with high precision, specificity and sensitivity.

The prenatal screening method object of the present description, being a test based on the application of combined molecular analyzes, Next Generation Sequencing, Digital PCR, Real Time PCR, ARMS, Dot Blot, drastically reduces the percentage share of false positives (<0.1%), and increases the diagnostic power of the NIPT test.

The method according to the present invention allows the study of the aforementioned chromosomal anomalies by analyzing maternal blood samples and detecting fetal aneuploidies by means of next-generation sequencing (NGS, Next Generation Sequencing), applying the steps described for the method and confirming the positive results with Digital PCR. The test, offered to pregnant women who have passed the 10th week of gestation, allows them to limit the use of invasive prenatal examinations (amniocentesis and villocentesis) resorting to such maneuvers when necessary. At the request of the patient, it is possible to search for fetal HR by Real Time PCR, while with the ARMS and the Dot Blot technique the main mutations of the CFTR gene associated with Cystic Fibrosis are analyzed.

## Claims

1. Method for determining the probability of the risk of chromosomal and genetic disorders from free DNA of fetal origin comprising the following steps:
- extracting said free DNA of fetal origin from a sample of maternal plasma previously collected;
- sequencing said free DNA of fetal origin extracted;
- obtaining the sequencing data in FASTQ format comprising the biological sequences and the quality scores of said biological sequences;
said method being **characterized in that** it comprises the following steps:
- converting said sequencing from said FASTQ format to the SAM format, in which said biological sequences are aligned with a reference genome;
- converting said sequencing from the SAM format to the BAM format, in which the data contained in said SAM format are compressed by conversion into binary code;
- counting and categorizing the READ sequenced from said SAM format, in which each READ is categorized as mapped on each chromosome or mapped on all autosomes;
- defining a predetermined reference sample;
- calculating the z-score on a chromosome of interest with respect to said predetermined reference sample;
determining the probability of anomaly risk on said chromosome of interest if said z-score is higher than a predetermined value.

2. Method for determining the probability of the risk of chromosomal and genetic disorders from free DNA of fetal origin according to claim 1, wherein the method further comprises the steps of:
- defining a set of TILES genomic regions that make up the genome in BAM format, in which the data are compressed by conversion into binary code;
- defining a reference genomic region in BAM format, in which the data are compressed by conversion into binary code, in which the parameters for implementing said BAM format of said reference genomic region are the same as said BAM format of said TILES genomic regions;
- comparing said BAM format of said TILES genomic regions with said BAM format of said reference genomic region;
- determining the probability of microdeletion or microduplication risk on said TILES genomic regions that make up the genome.

3. Method for determining the probability of the risk of chromosomal and genetic disorders from free DNA of fetal origin according to claim 1 or 2, wherein said method further comprises the step of defining a BAI index format from said compressed BAM format, in which said BAI index format is adapted to the execution of each software package that works on said compressed BAM format.

4. Method for determining the probability of the risk of chromosomal and genetic disorders from free DNA of fetal origin according to one or more of claims 1 to 3, wherein said step of converting said sequencing from said FASTQ format to the SAM format, in which said biological sequences are aligned with a reference genome, is implemented by the Burrows-Wheeler algorithm.

5. Method for determining the probability of the risk of chromosomal and genetic disorders from free DNA of fetal origin according to one or more of claims 1 to 4, wherein said step of sequencing said free DNA of fetal origin extracted is carried out by means of Next Generation Sequencing NGS techniques.

6. Method for determining the probability of the risk of chromosomal and genetic disorders from free DNA of fetal origin according to one or more of claims 1 to 5, wherein said method further comprises the step of amplifying said free DNA of fetal origin by Polymerase Chain Reaction PCR, in which said amplification step is carried out downstream of said step of extracting said free DNA of fetal origin and upstream of said step of sequencing said free DNA of fetal origin extracted.

7. Method for determining the probability of the risk of chromosomal and genetic disorders from free DNA of fetal origin according to one or more of claims 1 to 6, wherein said method further comprises the step of verifying said free DNA of fetal origin by DigitalPCR if in said step of determining the probability of anomaly risk on said chromosome of interest, said predetermined value is exceeded by said z-score.

8. Method for determining the probability of the risk of chromosomal and genetic disorders from free DNA of fetal origin according to one or more of claims 1 to 7, wherein said step of calculating said z-score on a chromosome of interest with respect to said predetermined reference sample is carried out on chromosomes 13, 18 and 21 for the determination of trisomies of the aforementioned chromosomes and/or for the determination of fetal sex and aneuploidy of the sex chromosomes X, Y and/or for the determination of the trisomies of said autosomes relative to one or more of chromosomes 1, 2, 3, 4, 5, 6, 7, 8, 9, 10, 11, 12, 14, 15, 16, 17, 19, 20, 22.

9. Method for determining the probability of the risk of chromosomal and genetic disorders from free DNA of fetal origin according to one or more of claims 2 to 7, wherein said step of determining the probability of risk of microdeletion or microduplication on said TILES genomic regions that make up the genome is related to one or more of Wolf-Hirschoom Syndrome, Jacobsen Syndrome, 1p36 deletion syndrome, Angelman Syndrome, DiGeorge Syndrome, Cri-du-chat Syndrome, Langer-Giedion Syndrome, Smith-Magenis Syndrome, Prader-Willi Syndrome, Williams Syndrome, Koolen-de-Vries Syndrome, HNPP Syndrome, 18q deletion syndrome, WAGR Syndrome, Potocki-Shaffer Syndrome, 1q21.1 deletion syndrome, Kleefstra, Phelan-Mcdermid Syndrome, Rubistein-Taybi Syndrome, Alagille Syndrome and Miller-Dieker Syndrome.

10. Method for determining the probability of the risk of chromosomal and genetic disorders from free DNA of fetal origin according to one or more of claims 2 to 7, wherein said step is obtained for determining the probability of risk of
1. Cystic fibrosis (CFTR GENE)
2. Congenital deafness (GJB2 GENE)
3. Beta thalassemia (HBB GENE)
4. Classical congenital adrenal hyperplasia due to 21-hydroxylase deficiency (CYP21A2 GENE)
5. Hemochromatosis (HFE GENE)
6. Achondroplasia (FGFR3 GENE)
7. Hypochondroplasia (FGFR3 GENE)
8. Dysatophor dysplasia (FGFR3 GENE)
9. Apert syndrome (FGFR2 GENE)
10. Crouzon syndrome (FGFR2 GENE)
11. Leopard syndrome (PTPN11 GENE)
12. Noonan syndrome (PTPN11/SOS1/RAF1 GENES)
13. Phenylketonuria (PAH GENE)
14. Rett syndrome (MECP2 GENE)
15. Recessive polycystic kidney (PKHD1 GENE).

## Patentansprüche

1. Verfahren zum Bestimmen der Wahrscheinlichkeit des Risikos von chromosomalen und genetischen Störungen aus freier DNA fetalen Ursprungs, das die folgenden Schritte aufweist:
- Extrahieren der freien DNA fetalen Ursprungs aus einer zuvor entnommenen Probe mütterlichen Plasmas;
- Sequenzieren der extrahierten freien DNA fetalen Ursprungs;
- Erhalten der Sequenzierungsdaten im FASTQ-Format, die die biologischen Sequenzen und die Qualitätsbewertungen der biologischen Sequenzen aufweisen; wobei das Verfahren **dadurch gekennzeichnet ist, dass** es die folgenden Schritte aufweist:
- Umwandeln der Sequenzierung vom FASTQ-Format in das SAM-Format, in dem die biologischen Sequenzen an ein Referenzgenom angeglichen werden;
- Umwandeln der Sequenzierung vom SAM-Format in das BAM-Format, in dem die in dem SAM-Format enthaltenen Daten durch Umwandlung in Binärcode komprimiert werden;
- Zählen und Kategorisieren des aus dem SAM-Format sequenzierten READs, wobei jedes READ als auf jedem Chromosom abgebildet oder als auf allen Autosomen abgebildet kategorisiert wird;
- Definieren einer vorgegebenen Referenzprobe;
- Berechnen des z-Scores auf einem interessierenden Chromosom in Bezug auf die vorgegebene Referenzprobe;
Bestimmen der Wahrscheinlichkeit eines Anomalierisikos auf dem interessierenden Chromosom, wenn der z-Score höher als ein vorgegebener Wert ist.

2. Verfahren zum Bestimmen der Wahrscheinlichkeit des Risikos von chromosomalen und genetischen Störungen aus freier DNA fetalen Ursprungs gemäß Anspruch 1, wobei das Verfahren weiterhin die folgenden Schritte aufweist:
- Definieren eines Satzes genomischer TILES-Gebiete, die das Genom bilden, im BAM-Format, wobei die Daten durch Umwandlung in Binärcode komprimiert werden;
- Definieren eines genomischen Referenzgebiets im BAM-Format, in dem die Daten durch Umwandlung in Binärcode komprimiert sind, wobei die Parameter zum Implementieren des BAM-Formats des genomischen Referenzgebiets dieselben sind wie das BAM-Format der genomischen TILES-Gebiete;
- Vergleichen des BAM-Formats der genomischen TILES-Gebiete mit dem BAM-Format des genomischen Referenzgebiets;
- Bestimmen der Wahrscheinlichkeit eines Mikrodeletions- oder Mikroduplikationsrisikos an den genomischen TILES-Gebieten, die das Genom bilden.

3. Verfahren zum Bestimmen der Wahrscheinlichkeit des Risikos von chromosomalen und genetischen Störungen aus freier DNA fetalen Ursprungs gemäß Anspruch 1 oder 2, wobei das Verfahren weiterhin den Schritt des Definierens eines BAI-Indexformats aus dem komprimierten BAM-Format aufweist, wobei das BAI-Indexformat an die Ausführung eines jeden Softwarepakets, das mit dem komprimierten BAM-Format arbeitet, angepasst ist.

4. Verfahren zum Bestimmen der Wahrscheinlichkeit des Risikos von chromosomalen und genetischen Störungen aus freier DNA fetalen Ursprungs gemäß einem oder mehr der Ansprüche I bis 3, wobei der Schritt des Umwandelns der Sequenzierung von dem FASTQ-Format in das SAM-Format, in dem die biologischen Sequenzen an einem Referenzgenom angeglichen werden, durch den Burrows-Wheeler-Algorithmus implementiert wird.

5. Verfahren zum Bestimmen der Wahrscheinlichkeit des Risikos von chromosomalen und genetischen Störungen aus freier DNA fetalen Ursprungs gemäß einem oder mehr der Ansprüche 1 bis 4, wobei der Schritt des Sequenzierens der extrahierten freien DNA fetalen Ursprungs durch Next-Generation-Sequencing NGS-Techniken durchgeführt wird.

6. Verfahren zum Bestimmen der Wahrscheinlichkeit des Risikos von chromosomalen und genetischen Störungen aus freier DNA fetalen Ursprungs gemäß einem oder mehr der Ansprüche 1 bis 5, wobei das Verfahren weiterhin den Schritt des Verstärkens der freien DNA fetalen Ursprungs durch Polymerase-Kettenreaktion ("Polymerase Chain Reaction"; PCR) aufweist, wobei der Verstärkungsschritt dem Schritt des Extrahierens der freien DNA fetalen Ursprungs nachgelagert und dem Schritt des Sequenzierens der extrahierten freien DNA fetalen Ursprungs vorgelagert durchgeführt wird.

7. Verfahren zum Bestimmen der Wahrscheinlichkeit des Risikos von chromosomalen und genetischen Störungen aus freier DNA fetalen Ursprungs gemäß einem oder mehr der Ansprüche 1 bis 6, wobei das Verfahren weiterhin den Schritt des Verifizierens der freien DNA fetalen Ursprungs durch DigitalPCR aufweist, wenn in dem Schritt des Bestimmens der Wahrscheinlichkeit des Anomalierisikos auf dem interessierenden Chromosom der vorgegebene Wert durch den z-Score überschritten wird.

8. Verfahren zum Bestimmen der Wahrscheinlichkeit des Risikos von chromosomalen und genetischen Störungen aus freier DNA fetalen Ursprungs gemäß einem oder mehr der Ansprüche 1 bis 7, wobei der Schritt des Berechnens des z-Scores auf einem Chromosom von Interesse in Bezug auf die vorgegebene Referenzprobe an den Chromosomen 13, 18 und 21 zur Bestimmung von Trisomien der vorgenannten Chromosomen und/oder zur Bestimmung des fetalen Geschlechts und der Aneuploidie der Geschlechtschromosomen X, Y und/oder zur Bestimmung der Trisomien der genannten Autosomen relativ zu einem oder mehr der Chromosomen 1, 2, 3, 4, 5, 6, 7, 8, 9, 10, 11, 12, 14, 15, 16, 17, 19, 20, 22 durchgeführt wird.

9. Verfahren zum Bestimmen der Wahrscheinlichkeit des Risikos von chromosomalen und genetischen Störungen aus freier DNA fetalen Ursprungs gemäß einem oder mehr der Ansprüche 2 bis 7, wobei der Schritt des Bestimmens der Wahrscheinlichkeit des Risikos einer Mikrodeletion oder Mikroduplikation auf den genomischen TILES-Gebieten, die das Genom bilden, mit einem oder mehr der folgenden Syndrome zusammenhängt: Wolf-Hirschhorn-Syndrom, Jacobsen-Syndrom, 1p36-Deletionssyndrom, Angelman-Syndrom, DiGeorge-Syndrom, Cri-du-chat-Syndrom, Langer-Giedion-Syndrom, Smith-Magenis-Syndrom, Prader-Willi-Syndrom, Williams-Syndrom, Koolen-deVries-Syndrom, HNPP-Syndrom, 18q-Deletionssyndrom, WAGR-Syndrom, Potocki-Shaffer-Syndrom, 1q21.1-Deletionssyndrom, Kleefstra, Phelan-Mcdermid-Syndrom, Rubinstein-Taybi-Syndrom, Alagille-Syndrom und Miller-Dieker-Syndrom.

10. Verfahren zum Bestimmen der Wahrscheinlichkeit des Risikos von chromosomalen und genetischen Störungen aus freier DNA fetalen Ursprungs gemäß einem oder mehr der Ansprüche 2 bis 7, wobei der Schritt zum Bestimmen der Wahrscheinlichkeit des Risikos für
1. Mukoviszidose (CFTR-GEN)
2. Angeborene Taubheit (GJB2-GEN)
3. Beta-Thalassämie (HBB-GEN)
4. Klassische angeborene Nebennierenhyperplasie aufgrund von 21-Hydroxylase-Mangel (CYP21A2-GEN)
5. Hämochromatose (HFE-GEN)
6. Achondroplasie (FGFR3-GEN)
7. Hypochondroplasie (FGFR3-GEN)
8. Dysatophor-Dysplasie (FGFR3-GEN)
9. Apert-Syndrom (FGFR2-GEN)
10. Crouzon-Syndrom (FGFR2-GEN)
11. Leopard-Syndrom (PTPN11-GEN)
12. Noonan-Syndrom (PTPN11/SOS1/RAF11l-GENE)
13. Phenylketonurie (PAH-GEN)
14. Rett-Syndrom (MECP2-GEN)
15. Rezessive polyzystische Niere (PKHD1-GEN)
erhalten wird.

## Revendications

1. Procédé de détermination de la probabilité du risque de troubles chromosomiques et génétiques à partir d'ADN libre d'origine foetale comprenant les étapes suivantes :
- l'extraction dudit ADN libre d'origine foetale à partir d'un échantillon de plasma maternel précédemment prélevé ;
- le séquençage dudit ADN libre d'origine foetale extrait ;
- l'obtention des données de séquençage au format FASTQ comprenant les séquences biologiques et les scores de qualité desdites séquences biologiques ;
ledit procédé de fabrication étant **caractérisé en ce qu'**il comprend les étapes suivantes :
- la conversion dudit séquençage dudit format FASTQ au format SAM, dans lequel lesdites séquences biologiques sont alignées sur un génome de référence ;
- la conversion dudit séquençage du format SAM au format BAM, dans lequel les données contenues dans ledit format SAM sont compressées par conversion en code binaire ;
- le comptage et la catégorisation de la LECTURE séquencée à partir dudit format SAM, dans lequel chaque LECTURE est catégorisée comme étant cartographiée sur chaque chromosome ou cartographiée sur tous les autosomes ;
- la définition d'un échantillon de référence prédéterminé ;
- le calcul du score z sur un chromosome d'intérêt par rapport audit échantillon de référence prédéterminé ;
la détermination de la probabilité de risque d'anomalie sur ledit chromosome d'intérêt si ledit score z est plus élevé qu'une valeur prédéterminée.

2. Procédé de détermination de la probabilité du risque de troubles chromosomiques et génétiques à partir d'ADN libre d'origine foetale selon la revendication 1, dans lequel le procédé comprend en outre les étapes suivantes :
- la définition d'un ensemble de régions génomiques de type MOSAÏQUE qui constituent le génome au format BAM, dans lequel les données sont compressées par conversion en code binaire ;
- la définition d'une région génomique de référence au format BAM, dans lequel les données sont compressées par conversion en code binaire, dans lequel les paramètres de mise en œuvre dudit format BAM de ladite région génomique de référence sont identiques à ceux dudit format BAM desdites régions génomiques de type MOSAÏQUE ;
- la comparaison dudit format BAM desdites régions génomiques de type MOSAÏQUE audit format BAM de ladite région génomique de référence ;
- la détermination de la probabilité du risque de microdéletion ou de microduplication sur lesdites régions génomiques de type MOSAÏQUE qui constituent le génome.

3. Procédé de détermination de la probabilité du risque de troubles chromosomiques et génétiques à partir d'ADN libre d'origine foetale selon la revendication 1 ou 2, dans lequel ledit procédé comprend en outre l'étape de définition d'un format d'index BAI à partir dudit format BAM compressé, dans lequel ledit format d'index BAI est adapté à l'exécution de chaque progiciel qui fonctionne sur ledit format BAM compressé.

4. Procédé de détermination de la probabilité du risque de troubles chromosomiques et génétiques à partir d'ADN libre d'origine foetale selon une ou plusieurs des revendications 1 à 3, dans lequel ladite étape de conversion dudit séquençage dudit format FASTQ au format SAM, dans lequel lesdites séquences biologiques sont alignées sur un génome de référence, est mise en œuvre par l'algorithme de Burrows-Wheeler.

5. Procédé de détermination de la probabilité du risque de troubles chromosomiques et génétiques à partir d'ADN libre d'origine foetale selon une ou plusieurs des revendications 1 à 4, dans lequel ladite étape de séquençage dudit ADN libre d'origine foetale extrait est effectuée au moyen de techniques de séquençage de nouvelle génération (NGS).

6. Procédé de détermination de la probabilité du risque de troubles chromosomiques et génétiques à partir d'ADN libre d'origine foetale selon une ou plusieurs des revendications 1 à 5, dans lequel ledit procédé comprend en outre l'étape d'amplification dudit ADN libre d'origine foetale par réaction de polymérisation en chaîne (PCR), dans lequel ladite étape d'amplification est effectuée en aval de ladite étape d'extraction dudit ADN libre d'origine foetale et en amont de ladite étape de séquençage dudit ADN libre d'origine foetale extrait.

7. Procédé de détermination de la probabilité du risque de troubles chromosomiques et génétiques à partir d'ADN libre d'origine foetale selon une ou plusieurs des revendications 1 à 6, dans lequel ledit procédé comprend en outre l'étape de vérification dudit ADN libre d'origine foetale par PCR digitale si, lors de l'étape de détermination de la probabilité de risque d'anomalie sur ledit chromosome d'intérêt, ladite valeur prédéterminée est dépassée par ledit score z.

8. Procédé de détermination de la probabilité du risque de troubles chromosomiques et génétiques à partir d'ADN libre d'origine foetale selon une ou plusieurs des revendications 1 à 7, dans lequel ladite étape de calcul dudit score z sur un chromosome d'intérêt par rapport audit échantillon de référence prédéterminé est effectuée sur les chromosomes 13, 18 et 21 pour la détermination de trisomies des chromosomes susmentionnés et/ou pour la détermination du sexe foetal et de l'aneuploïdie des chromosomes sexuels X, Y et/ou pour la détermination des trisomies desdits autosomes par rapport à un ou plusieurs des chromosomes 1, 2, 3, 4, 5, 6, 7, 8, 9, 10, 11, 12, 14, 15, 16, 17, 19, 20, 22.

9. Procédé de détermination de la probabilité du risque de troubles chromosomiques et génétiques à partir d'ADN libre d'origine foetale selon une ou plusieurs des revendications 2 à 7, dans lequel ladite étape de détermination de la probabilité du risque de microdélétion ou de microduplication sur lesdites régions génomiques de type MOSAÏQUE qui constituent le génome est liée à un ou plusieurs éléments parmi le syndrome de Wolf-Hirshhorn, le syndrome de Jacobsen, le syndrome de délétion 1p36, le syndrome d'Angelman, le syndrome de DiGeorge Syndrome, le syndrome du cri du chat, le syndrome de Langer-Giedion, le syndrome de Smith-Magenis, le syndrome de Prader-Willi, le syndrome de Williams, le syndrome de Koolen-de-Vries, la neuropathie héréditaire avec hypersensibilité à la pression (HNPP), le syndrome de délétion 18q, le syndrome WAGR, le syndrome de Potocki-Shaffer, le syndrome de la délétion 1q21.1, le syndrome de Kleefstra, le syndrome de Phelan-McDermid, le syndrome de Rubinstein-Taybi, le syndrome d'Alagille et le syndrome de Miller-Dieker.

10. Procédé de détermination de la probabilité du risque de troubles chromosomiques et génétiques à partir d'ADN libre d'origine foetale selon une ou plusieurs des revendications 2 à 7, dans lequel ladite étape est obtenue pour la détermination de la probabilité du risque de
1. Fibrose kystique (GÈNE CFTR)
2. Surdité congénitale (GÈNE GJB2)
3. Bêta-thalassémie (GÈNE HBB)
4. Hyperplasie surrénale congénitale classique due à un déficit en 21-hydroxylase (GÈNE CYP21A2)
5. Hémochromatose (GÈNE HFE)
6. Achondroplasie (GÈNE FGFR3)
7. Hypochondroplasie (GÈNE FGFR3)
8. Dysplasie dysatophore (GÈNE FGFR3)
9. Syndrome d'Apert (GÈNE FGFR2)
10. Syndrome de Crouzon (GÈNE FGFR2)
11. Syndrome LEOPARD (GÈNE PTPN11)
12. Syndrome de Noonan (GÈNES PTPN11/SOS1/RAF1)
13. Phénylcétonurie (GÈNE PAH)
14. Syndrome de Rett (GÈNE MECP2)
15. Polykystose rénale récessive (GÈNE PKHD1).
